# EUROPÄISCHE PATENTANMELDUNG

(11) **EP 0 521 347 A1**
(43) Veröffentlichungstag der Anmeldung: **07.01.1993**
(21) Anmeldenummer: 92110243.0
(22) Anmeldetag: 17.06.1992
(51) Int. Cl.: C08G 65/32, C07C 41/18, C07C 43/12

(54) **Verfahren zur Herstellung von perfluorierten Ethern**

(30) Priorität: 21.06.1991 DE 4120508
(71) Anmelder: HOECHST AKTIENGESELLSCHAFT, 65926 Frankfurt am Main (DE)
(72) Erfinder: Meyer, Matthias, Dr., W-6230 Frankfurt am Main 80 (DE)

(57) **Zusammenfassung**

Die Erfindung betrifft ein Verfahren zur Herstellung von perfluorierten Ethern der Formel R - CF₂ - CF₃, worin R den Rest CF₃(CF₂)₂-O-[-CF(CF₃)CF₂-O]ₙ- bedeutet, in dem n eine ganze Zahl von 0-60 ist, durch Decarbonylierung von Perfluoretheracylfluoriden der Formel R - CF(CF₃)-COF bei 150-350 °C in Gegenwart von AlCl₃ und/oder AlBr₃.

## Beschreibung

Die Erfindung betrifft ein Verfahren zur Herstellung von perfluorierten Ethern der Formel (I)

R - CF₂- CF₃ (I)

worin R den Rest CF₃(CF₂)₂-O-[-CF(CF₃)CF₂-O]ₙ- bedeutet, in dem n eine ganze Zahl von 0-60 ist, durch Decarbonylierung von Perfluoretheracylfluoriden der Formel R - CF(CF₃) - COF.

Perfluorether der Formel (I) zeichnen sich durch eine hohe thermische Beständigkeit aus, sie sind unbrennbar und chemisch stabil, auch gegenüber Angriffen starker Oxydationsmittel.

Aufgrund ihrer besonderen physikalischen Eigenschaften, nämlich günstigem Viskositätsverhalten in weiten Temperaturbereichen, hohen Siedepunkten bei gleichzeitig niedrigen Stockpunkten, geringer Oberflächenspannung und extremen dielektrischen Eigenschaften, finden perfluorierte Polyether als Inertflüssigkeiten, Dielektrika, Wärmetauschmittel, Hydrauliköle und Schmiermittel in Gegenwart von stark aggressiv wirkenden Medien, vielseitige Anwendungen.

In EP-A-0 154 297 ist die Oligomerisation von Hexafluorpropenoxid (HFPO) in Gegenwart von CsF und Polyethylenglykoldimethylethern, insbesondere Tetraethylenglykoldimethylether, beschrieben. Das Roholigomer fällt dabei im allgemeinen als Gemisch mit einer Molmassenverteilung von ca. 300-20 000 g/mol an. Es besteht aus Perfluoretheracylfluoriden der Formel R-CF(CF₃)COF, wobei R die oben angegebene Bedeutung hat, die Verbindungen sind aber noch mit CsF und dem als Lösungsmittel eingesetzten Polyethylenglykoldimethylether verunreinigt.

Die genannten Acylfluoride sind hydrolyseempfindlich, spalten bei hoher Temperatur aggressive und toxische fluorierte Bruchstücke ab und sind daher als Inertflüssigkeiten nicht einsetzbar.

Verschiedene Methoden zur "Endgruppenstabilisierung" dieser Acylfluoride, d.h. Umwandlung in perfluorierte Ether sind bekannt.

Gemäß EP-A-0 154 297 werden die Acylfluoride zu Carbonsäuren hydrolysiert, in die Salze überführt und diese anschließend in Gegenwart von alkalischen Medien decarboxyliert. Auf diesem Wege erhält man jedoch nur unvollständig fluorierte Ether der Formel R-CHFCF₃.

Es ist ferner aus US-PS 3 242 218 bekannt, daß die den Acylfluoriden entsprechenden Carbonsäuren mit elementarem Fluor decarboxylierend fluoriert werden können. Dabei wird die Carboxylgruppe gemäß der Gleichung

R - CF(CF₃)COOH + F₂ --- > R - CF₂CF₃ + CO₂ + HF

durch ein Fluoratom ersetzt. Bei diesem Verfahren ist es erforderlich, die gemäß EP-A-0 154 297 erhältlichen Acylfluoride zunächst zu verseifen und die hergestellten Carbonsäuren sorgfältig zu trocknen, bevor die Umsetzung mit Fluor vorgenommen wird. Sowohl bei der Verseifung, als auch bei der Fluorierung fällt Fluorwasserstoff als Nebenprodukt an.

In DE-OS 2 451 493 wird die Direktfluorierung der Acylfluoride mit elementarem Fluor zu perfluorierten Ethern in Gegenwart von Ag/Cu-Katalysatoren nach folgender Gleichung beschrieben:

R - CF(CF₃)COF + F₂ --- > R - CF₂CF₃ + COF₂.

Nach US-PS 3 555 100 werden die Acylfluoride direkt- ohne vorherige Verseifung - in Gegenwart von Antimonpentafluorid zu Perfluorethern der Formel (I) decarbonyliert. Nebenreaktionen mit Antimonpentafluorid führen jedoch zu Fragmentierungsprodukten, die die Ausbeute vermindern. Die Verweilzeit der Reaktionspartner ist besonders bei höheren Temperaturen kritisch und möglichst kurz zu halten. Außerdem müssen nach der Umsetzung Antimonpentafluorid und seine in der Nebenreaktion gebildeten Folgeprodukte abgetrennt werden. Dies erfordert wäßrige Aufarbeitung mit Zerstörung des wertvollen SbF₅-Katalysators. Darüber hinaus entstehen Abwasserprobleme und die Perfluorether müssen außerdem getrocknet werden.

In Chemical Abstracts Vol. 95 (1981) 25879t wird beschrieben, daß die Acylfluoride mit gleichen Gewichtsanteilen AlF₃ bei hohen Temperaturen zu den Perfluorethern der Formel (I) decarbonyliert werden können. Ein Nachteil dieses Verfahrens ist der hohe Einsatz an AlF₃. Bei den beschriebenen Reaktionstemperaturen von 300-320 °C sind außerdem Fragmentierungsreaktionen zu erwarten. Eine solche Fragmentierung von Acylfluoriden der Formel R-CF(CF₃)COF mit AlF₃ ist in EP-A-0 167 258 beschrieben.

Gegenstand der Erfindung ist ein Verfahren zur Herstellung von perfluorierten Ethern der Formel (I)

R - CF₂ - CF₃ (I)

worin R den Rest CF₃(CF₂)₂-O-[-CF(CF₃)CF₂-O]ₙ- bedeutet, in dem n eine ganze Zahl von 0-60 ist, durch Decarbonylierung von Perfluoretheracylfluoriden der Formel R - CF(CF₃)-COF, worin R die genannte Bedeutung hat, dadurch gekennzeichnet, daß man die Decarbonylierung bei 150-350 °C in Gegenwart von AlCl₃ und/oder AlBr₃ durchführt.

Die Acylfluoride werden beispielsweise in einem Rührkolben unter Inertgasatmosphäre mit AlCl₃ und/oder AlBr₃ auf Reaktionstemperatur erhitzt. Vorzugsweise legt man jedoch das Acylfluorid vor, erhitzt es, und fügt dann AlCl₃ und/oder AlBr₃ hinzu. Ein Lösungsmittel ist nicht erforderlich.

Die zugesetzten Mengen an AlCl₃ und/oder AlBr₃ betragen im allgemeinen 1-100 Mol-%, bezogen auf eingesetztes Acylfluorid, vorzugsweise 3-10 Mol-%.

Die Reaktionstemperatur beträgt 150-350 °C, vorzugsweise 200-280 °C, insbesondere 240-260 °C.

Es werden Acylfluoride der allgemeinen Formel R-CF(CF₃)COF eingesetzt, wobei n eine ganze Zahl von 0 bis 60 ist. Der Wert von n kann durch die Temperatur bei der Oligomerisation von HFPO gemäß EP-A-0 154 297 gesteuert werden. Je tiefer die Temperatur gewählt wird, umso höher ist der Wert von n. Besonders interessant sind Werte von n=10 bis n=60. Es entsteht aber stets ein Gemisch von Acylfluoriden. Im allgemeinen setzt man beim erfindungsgemäßen Verfahren ein solches Gemisch ein. Man kann aber auch zuerst daraus ein einzelnes Acylfluorid durch Rektifikation isolieren und dann dieses einsetzen.

Vor dem Einsatz der Acylfluoride im erfindungsgemäßen Verfahren wird zunächst das gemäß EP-A-0 154 297 erhaltene HFPO-Roholigomer von dem darstellungsbedingt darin enthaltenen Cäsiumfluorid und Tetraethylenglykoldimethylether befreit, da diese Verbindungen mit den Aluminiumhalogeniden unerwünschte Nebenreaktionen eingehen können. Diese Reinigung wird durch Extraktion mit einem apolaren, aprotischen Lösungsmittel, anschließende Phasentrennung und Abfiltrieren des CsF von der Acylfluorid-Phase erreicht.

Die Umsetzung der Acylfluoride mit AlCl₃ und/oder AlBr₃ kann IR- und ¹⁹F-NMR-spektroskopisch verfolgt werden und ist beendet, wenn im IR-Spektrum die Carbonylbande bei etwa 1890 cm⁻¹ verschwunden ist und im ¹⁹F-NMR-Spektrum die Signale der CF(CF₃)COF-Endgruppe der Acylfluoride nicht mehr sichtbar sind.

Nach Reaktionsende wird das Produkt durch Filtration vom AlCl₃ und/oder AlBr₃-Feststoff abgetrennt. Das wasserklare, farblose Filtrat kann ohne weitere Reinigungsschritte der Fraktionierung in Siedebereiche durch Kurzwegdestillation zugeführt werden.

### Beispiele

### Versuchsbericht

Darstellung der Acylfluoride der Formel R-CF(CF₃)COF gemäß EP-A-0 154 297.

Eine Lösung von 20 g CsF in 50 ml Tetraethylenglykoldimethylether (Tetraglyme) und 56 g Hexafluorpropylenoxid wurden unter Stickstoffatmosphäre in einem 4 l V-4-A Autoklaven vorgelegt. Zusätzlich wurden 250 ml ®Frigen F113 zur Verdünnung hinzugefügt. Bei einer Temperatur von -5 °C wurden dann 4000 g Hexafluorpropylenoxid über einen Zeitraum von 8 Stunden unter guter Durchmischung eingeleitet. Nach Reaktionsende wurde auf Raumtemperatur erwärmt und das Roholigomer unter N₂-Atmosphäre abgelassen. Das vom Tetraglyme und CsF (durch Extraktion mit n-Hexan, anschließende Trennung der das Tetraglyme enthaltenden Hexan-Phase von der Acylfluorid-Phase und Abfiltrieren des CsF von der Acylfluorid-Phase) befreite Acylfluorid-Gemisch wurde anschließend in Beispiel 1 eingesetzt. Analog wurden Acylfluorid-Gemische bei Temperaturen von -8 °C bzw. + 5 °C für die Beispiele 2 bzw. 3 und 4 hergestellt.

### Beispiel 1

Es wurden 580 g (= 0,18 M) eines Acylfluorid-Gemischs mit einem mittleren Molekulargewicht von 3200 g/mol nach Zusatz von 0,8 g wasserfreiem Aluminiumchlorid (0,006 M) unter N₂-Atmosphäre auf 250 °C erhitzt. Bei Erreichen einer Temperatur von 200 °C setzte leichte CO-Entwicklung ein. Die Reaktion wurde ¹⁹F-NMR- und IR-spektroskopisch verfolgt und war nach ca. 5 Stunden beendet. Nach Abtrennung vom Feststoff AlCl₃ durch Filtration, wurden 493 g Perfluorether der Formel (I) erhalten.

### Beispiel 2

3657 g (= 0,9 M) Acylfluorid-Gemisch mit einem mittleren Molekulargewicht von 4100 g/mol (bei -8 °C hergestellt) wurden unter N₂-Atmosphäre auf 300 °C erhitzt. Nach Erreichen dieser Temperatur wurden 12 g wasserfreies Aluminiumchlorid (0,09 M) portionsweise zugefügt. Nach jeder Zugabe von AlCl₃ setzte heftige Gasentwicklung ein. Die Reaktion wurde ¹⁹F-NMR- und IR-spektroskopisch verfolgt und war nach 3 h beendet. Nach Filtration wurden 3330 g Produkt der Formel (I) mit einem mittleren Molekulargewicht von 3900 g/mol erhalten.

### Beispiel 3

1900 g (= 1,3 M) eines Acylfluorid-Gemischs mit einem mittleren Molekulargewicht von 1460 g/mol (bei + 5°C hergestellt) wurden mit 0,15 M wasserfreiem AlCl₃ versetzt und auf 225 °C erhitzt. Die Reaktion wurden spektroskopisch verfolgt und war nach 3 h beendet. Nach Filtration wurden 1320 g Produkt der Formel (I) mit einem mittleren Molekulargewicht von 1250 g/mol erhalten.

### Beispiel 4

Es wurde gearbeitet wie im Beispiel 3, jedoch wurden 35 g (= 0,13 M) Aluminiumbromid (wasserfrei) statt AlCl₃ zugefügt. Die Reaktion war nach 3,5 h beendet, und nach Filtration wurden 1480 g Produkt der Formel (I) mit einem mittleren Molekulargewicht von 1300 g/mol erhalten.

### Beispiel 5

125 g (0,15 M) eines Acylfluorids der Formel R-CF(CF₃)COF mit n=3 (erhalten durch Destillation eines bei + 5 °C hergestellten Acylfluorid-Gemischs) wurden mit 2 g AlCl₃ (= 0,015 M) versetzt und bis zum Siedepunkt (195 °C) erhitzt. Nach zweistündiger Reaktionszeit wurde aus dem Reaktionsgemisch über eine Kolonne bei 187 °C 60 g einer leichtbeweglichen Flüssigkeit abdestilliert, die gemäß ¹⁹F-NMR-, IR- und GC-Analyse die Formel (I) mit n=3 hatte. Außerdem wurden unter vermindertem Druck bei 100 °C/13 mm Hg 50 g der Verbindung R-CF(CF₃)COCl mit n=3 überdestilliert.

## Patentansprüche

1. Verfahren zur Herstellung von perfluorierten Ethern der Formel (I)
R - CF₂ - CF₃ (I)
worin R den Rest CF₃(CF₂)₂-O-[-CF(CF₃)CF₂-O]ₙ- bedeutet, in dem n eine ganze Zahl von 0-60 ist, durch Decarbonylierung von Perfluoretheracylfluoriden der Formel R - CF(CF₃)-COF, worin R die genannte Bedeutung hat, dadurch gekennzeichnet, daß man die Decarbonylierung bei 150-350 °C in Gegenwart von AlCl₃ und/oder AlBr₃ durchführt.

2. Verfahren nach Anspruch 1, dadurch gekennzeichnet, daß man 1-100 Mol-% AlCl₃ und/oder AlBr₃ verwendet, bezogen auf eingesetztes Perfluoretheracylfluorid.

3. Verfahren nach Anspruch 1, dadurch gekennzeichnet, daß man 3-10 Mol-% AlCl₃ und/oder AlBr₃ verwendet, bezogen auf eingesetztes Perfluoretheracylfluorid.

4. Verfahren nach einem der Ansprüche 1 bis 3, dadurch gekennzeichnet, daß man die Decarbonylierung bei 200-280 °C durchführt.

5. Verfahren nach einem der Ansprüche 1 bis 3, dadurch gekennzeichnet, daß man die Decarbonylierung bei 240-260 °C durchführt.
